# EUROPEAN PATENT SPECIFICATION

(11) **EP 1 474 197 B1**
(45) Date of publication and mention of the grant of the patent: **30.04.2008**
(21) Application number: 03705583.7
(22) Date of filing: 29.01.2003
(51) Int. Cl.: A61M 15/00, B05D 5/08, B05D 7/24, C08J 7/18

(54) **POLYACRYLATE COATED INHALATION DEVICE**
MIT POLYACRYLAT ÜBERZOGENES INHALIERGERÄT
DISPOSITIF D'INHALATION AYANT UN REVETEMENT AU RESINE POLYACRYLIQUE

(30) Priority: 01.02.2002 SE 0200313
(43) Date of publication of application: 10.11.2004
(73) Proprietor: AstraZeneca AB, 151 85 Södertälje (SE)
(72) Inventor: BADYAL, Jas Pal, University of Durham, Durham, Durham DH1 3LE (GB); ROGUEDA, Philippe, AstraZeneca R & D Charnwood, Loughborough, Leicestershire LE11 5RH (GB)
(74) Representative: Summersell, Richard John
(86) International application number: PCT/SE2003/000158
(87) International publication number: WO 2003/063938

(56) References cited:
- WO-A1-98/58117
- WO-A1-99/42154
- US-A- 5 932 299

## Description

### Field of the invention:

This invention describes how cold plasma coating can be used to improve the performance of inhalation devices. In particular, it describes the use of fluorinated acrylate molecules to reduce drug adhesion to device components. These compounds conjointly with the way they are grafted onto surfaces are new to the area of inhalation devices. This invention can be applied to a range of surfaces (metals, plastics) and used with a range of drug molecules for the treatment of local conditions (mouth, lung, nose, throat and lung diseases). It can also be used in the systemic treatment a wide range of ailments (lung cancer, migraine, diabetes etc...).

### Background of the invention:

Inhalation treatments of a series of medical conditions rely on sophisticated devices to deliver the drug to the desired target. These devices can themselves introduce new variables in the optimisation of the product performance. These are mainly due to drug adsorption to the elements constituting the device. For example, the device can be a pMDI used in the treatment of respiratory diseases. A pMDI is made up of a canister, an actuator and a complex valve assembly. All the components of this pMDI are potential sites of drug adhesion, therefore loss of drug leading to irregular dosing. Reducing drug adhesion is of primary importance for the design of a robust product. This can be achieved by modifying the drug formulation or the surface properties of the device components.

The problem is particularly acute in pMDI. The requirement to use HFA propellants, for the treatment of asthma for instance, imposed by the Montreal protocol has led to a series of unexpected hurdles in the development of new products. This is mostly due to the characteristics of the liquid HFA and the behaviour of the drug suspensions prepared with them. HFA propellants have very low surface tensions and wet solid surfaces with remarkable efficiency. This means that due to capillary forces, the propellants spread up can walls very easily, and leave a thin film of caked particles. Furthermore, because of the poor solvent properties of the propellants, drug particles have a tendency to move to the propellant/device interface to have a more energetically favourable contact than they would by remaining in the bulk. Both phenomenon can be reduced by coating the device and its components with appropriate chemicals. A range of coatings can be applied in a variety of fashion with a varying degree of success.

One of the early forms of coating is spray coating. It is a process akin to painting and is used successfully in the food and paint industry. For example PCT/SE/01/01606 discloses the spray coating of fluorinated alkylpolyglycosides. Most spray coating methods suffer from the drawback to rely on multiple steps processes. The can has to be sprayed and dried before use. Anodisation of the aluminium parts is often required as well. The uniformity and integrity of such coating can be an issue, since spray guns cannot access the intricate details of some components design. Finally, the coating layer thus deposited can be scraped off the surface, and introduces leachables and unwanted chemicals in the medicament composition.

Silanisation is another way of modifying surface properties. For example PCT/SE/01/12749 indicates how such treatment can be used to reduce drug adhesion. This treatment is a solvent based method and require the cans to be washed after treatment and tested for cleanliness before being put to use for medical treatment.

Plasma coating is known in the art, for example WO 99/64662 and WO 00/05000 disclose plasma coating of a surface such as a fabric. GB 2 355 252 discloses plasma coating of a medicinal device with certain silicon based polymers. However Fluorinated acrylates are not disclosed nor suggested.

Reducing the attraction between drug particles and solid surfaces will lead to a reduce particle adhesion, since the particles will not reach the surfaces.

### Summary of the invention

It has been found that drug adhesion to the elements (i.e. can, stem, valves, seals etc...) of inhalation devices (nasal, nebulisers, pMDIs, DPIs...) can be dramatically reduced by coating these elements by cold plasma with fluorinated acrylates. The process can either be pulsed or continuous. Some or all of the above components can be coated using the processes of the invention.

In a first aspect, the invention provides a device for dispensing a drug by inhalation wherein the device or components thereof where drug adhesion can occur are coated by a cold plasma coating process characterised in that the coating is a fluorinated acrylate compound selected from 1H, 1H, 2H, 2H heptadecafluoradecyl acrylate, 1H, 1H, 2H, 2H perfluoroctyl acrylate or 1H, 1H, 2H, 2H perfluoroctyl methacrylate or a mixture thereof,

In a further aspect, the invention provides use of a fluorinated acrylate compound for the cold plasma coating of a medicinal device.

In a further aspect, the invention provides a process for coating a medicinal device with a fluorinated acrylate which comprises coating the device with a cold plasma.

The term "cold plasma" means that the temperature within the body of the plasma is ambient.

The advantages of the invention are that no spray guns are used for the coating. Henceforth, problems associated with conventional coating methods such as spray blockage can be avoided. In addition since the present invention employs cold plasma, polymers can be used that could not otherwise be used with conventional spraying such as unstable polymers The medicinal devices can be coated in a batch process. Cold plasma is also better than standard coating in that a more uniform coat is achieved and the coating can reach in the intricate part of the device design that standard spray coating fails to reach. Finally, a chemical bond is achieved between substracte and coating, which provides a longer lasting and more efficient coated layer.

The chemicals with which the device components can be coated are fluorinated acrylates suitable for plasma coating. Short chain acrylates are preferred.
The molecules can be straight of branched chains. Most preferably 1H, 1H, 2H, 2H heptadecafluorodecyl acrylate, 1H, 1H, 2H, 2H perfluoroctyl acrylate and 1H, 1H, 2H, 2H perfluoroctyl methacrylate can be used. Any other homologous compound in the acrylate family can also be used. This list does not pretend to be exhaustive, and any person skilled in the art could identify other compounds with similar structures as being covered in this invention. These would include homologous series of the previous molecules (i.e. with a varying chain length, or branching), and other straight chain fluorinated acrylates and methacrylates. Chain length less than 25 carbon atoms long are preferred, most preferably they should have less than 20 carbon atoms, most most preferably less than and including 16.

The thickness of the coated layer can range from I nm to 500 µm. Preferably between 5 nm and 100 µm.

The invention can be applied successfully to any part of an inhalation device where drug adhesion can occur. For pMDls, the actuator, the stem, the valve components, the measuring chamber, the seals, and the canister can all be treated. The invention includes also nebulisers, nasal sprays and dry powder inhalers (DPI's).

The invention is particularly successful with drugs suitable for the inhaled route.

Examples of specific drugs which can be used according to the invention include mometasone, ipratropium bromide, tiotropium and salts thereof, salemeterol, fluticasone propionate, beclomethasone dipropionate, reproterol, clenbuterol, rofleponide and salts, nedocromil, sodium cromoglycate, flunisolide, budesonide, formoterol fumarate dihydrate, Symbicort™ (budesonide and formoterol), 3-[2-(4-hydroxy-2-oxo-3H-1,3-benzothiazol-7-yl)ethylamino]-N-[2-[2-(4-methylphenyl)ethoxy)ethyl]propansulphonamide, terbutaline, terbutaline sulphate, salbutamol base and sulphate, fenoterol, 3-[2-(4-Hydroxy-2-oxo-3H-1,3-benzathiazol-7-yl)ethylamino]-N-[2-[2-(4-methylphenyl)ethoxy]ethyl]propanesulphonamide, hydrochloride. All of the above compounds can be in free base form or as pharmaceutically acceptable salts as known in the art.

The coating process can be carried out using the techniques described in WO 99/64662 and WO 00/05000.

The full potential of this invention is revealed for particles with a size distribution between 100 nm and 1000 µm, most preferably between 500 nm and 500 µm, even more preferably between 900 nm and 100 µm, most preferably between 900 nm and 30 µm.

The invention works particularly well when the particles are dispersed in a non-aqueous pressurised medium such as the propellants HFA227 and HFA134a, or any mixture thereof. The invention can also be applied successfully to devices having formulations with HFA propellants, or propellant mixtures to which have been added polymers or cosolvents.

The invention is illustrated by the following examples.

### Examples

To assess the usefulness of the coating technique for inhalation a series of tests were performed. Aluminium surfaces were coated with 3 types of polymers under 2 types of conditions. Interactions between drug particles and the treated surfaces were then measured by Atomic Force Microscopy (also known as AFM). The strength and range of these interactions were compared with the ones obtained from an uncoated surface.

The materials used to exemplify the invention were:
1H, 1H, 2H, 2H Heptadecafluorodecyl acrylate, 1H, 1H, 2H, 2H Perfluoroctyl Acrylate and 1H, 1H, 2H, 2H Perfluoroctyl Methacrylate.

The chemicals were cold plasma coated onto aluminium sheets. These Aluminium surfaces are the one currently used to manufacture pMDI canisters. With 3 different chemicals and 2 methods of production, a total of 6 coatings were investigated. In addition, an anodised aluminium surface and a non-anodised one were studied to provide a reference for the interactions.

The drug used in these tests was formoterol fumarate dihydrate (abbreviated as FFD). Its size distribution was centred around 2 µm.

The extent of the adhesion between the particles and the treated surfaces was tested in a fluorinated solvent, 2H,3H perfluoropentane (abbreviated as HPFP). This liquid is a very good substitute for both propellants HFA227 and HFA134a. It is used when tests can not be performed in situ in pressurised liquids, such as AFM.

Substrate surfaces were imaged in air with a Digital Instruments Nanoscope III AFM in TappingMode™ operation, using standard silicon TESP cantilevers (Nanoprobes, Digital Instruments, Santa Barbara). Typical scan rates for all images were between 1-1.5Hz with a pixel resolution of 512x512. Force-distance interactions and force volume data were recorded under in-situ conditions, via a hermetically sealed contact mode in-situ cell (Digital Instruments, Santa Barbara). Drug particles were glued on a tipless cantilever to act as a colloidal probe.

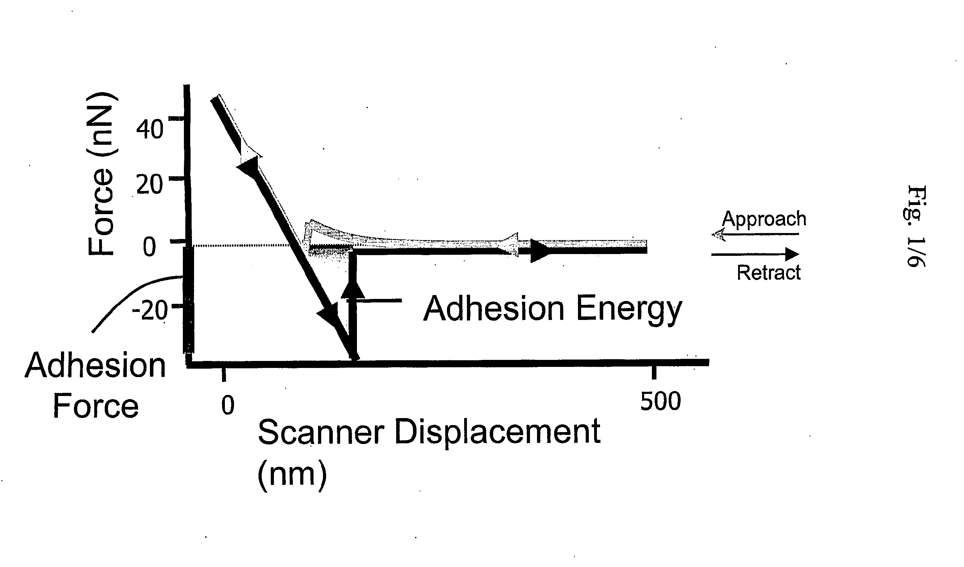

The adhesion force was calculated from the retracting force-distance curve. The adhesion energy is the product of the maximum force by the distance to which the cantilever retracts when going away from the surface (see figure 1). The AFM technique used in this study records a series of force-distance curves on a finite surface and for each points calculates an adhesion energy. When a repulsion is felt, no calculation is possible. The energy values quoted are average values over a finite element of the surface (on average 20 µm x 20 µm).

### Reference sample: non anodised aluminium surface:

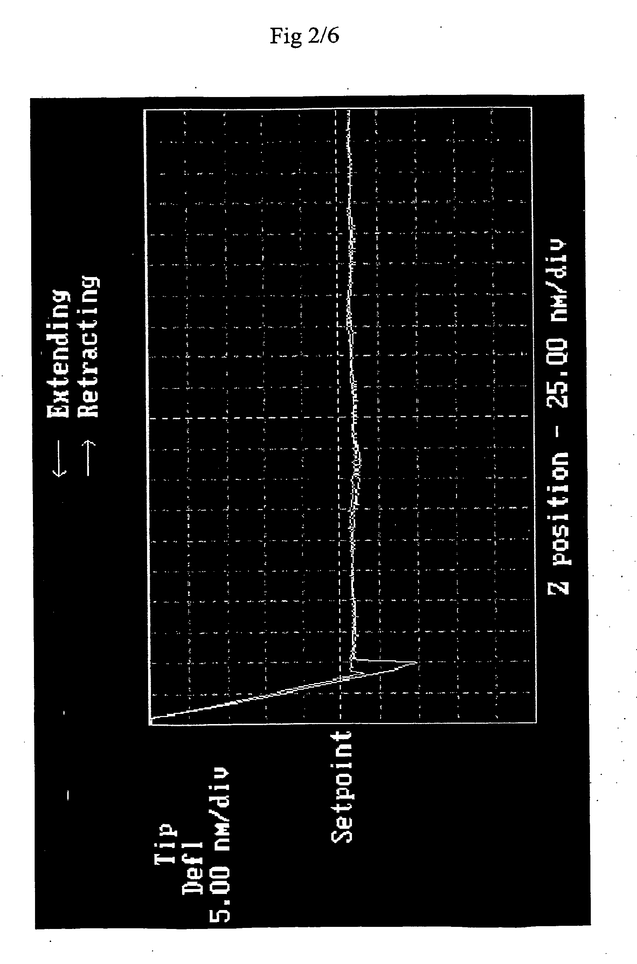

A non anodised aluminium surface was used as a reference. The force distance curve for this sample can be found on figure 2.

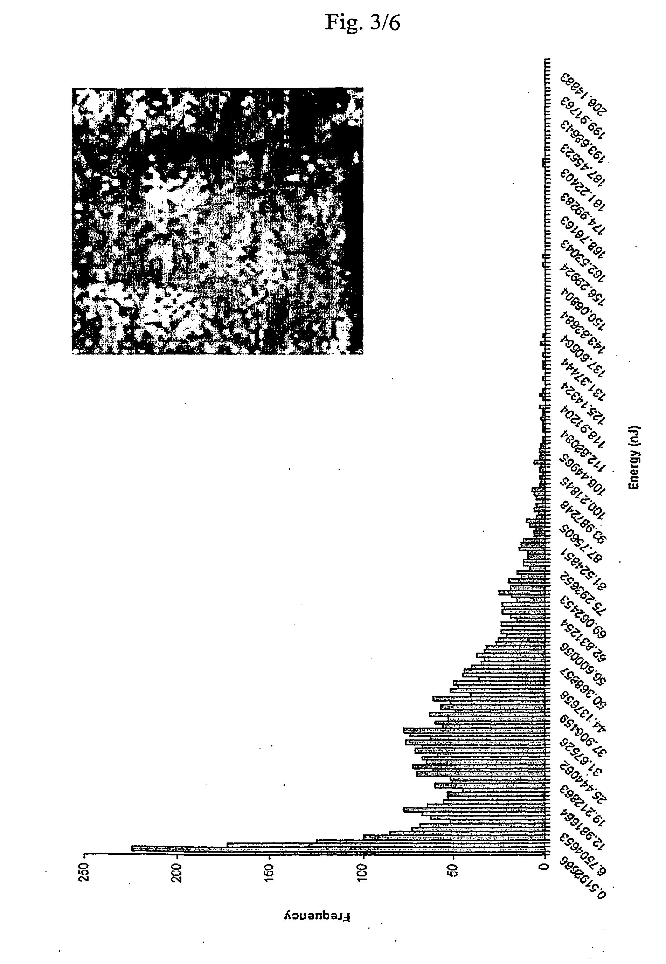

An attractive force was felt on approaching the surface. The attractive force led to an adhesion force on contact of ~ 8 nN. For each point measured an adhesion energy was calculated from the retraction experiment (i.e. when the cantilever is taken away from the surface). The adhesion energy spread can be found on figure 3. Because of the roughness of the surface, the adhesion energy spreads over a range of energy values. Two areas of adhesion are seen: a low adhesion centred around 5.8 nJ and a high adhesion region centred on 36.4 nJ. The energy spread is due to the variations in surface roughness.

### Reference sample: anodised aluminium surfaces:

A second reference sample was studied. This was an anodised aluminium surface. Anodised aluminium sheets are also used in the process of making pMDI canisters.

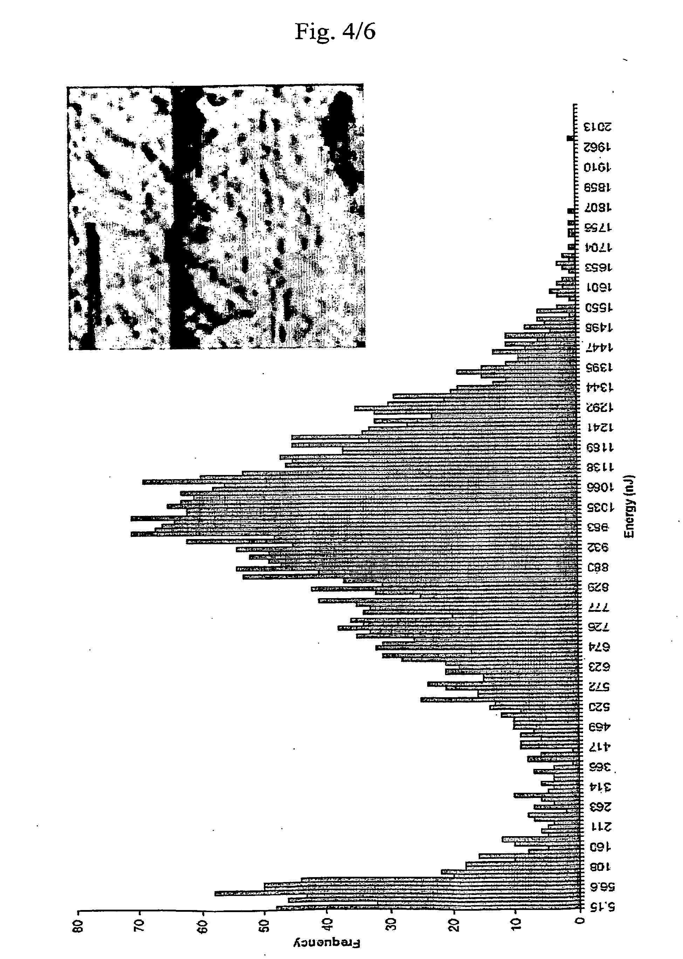

As with the previous sample, an attractive force was felt on approaching the drug particle to the surface. This attractive force was translated into and adhesive force on contact for which an adhesive energy can be calculated. The adhesion force was on average ~ 51.5 nN, yielding adhesion energies between 5 and 1500 nJ. (see figure 4 for energy diagram). The high adhesion energies are centred on 1038 nJ.

### Sample 1: aluminium surface cold plasma coated with 1H, 1H, 2H, 2H heptadecafluorodecyl acrylate with a continuous wave.

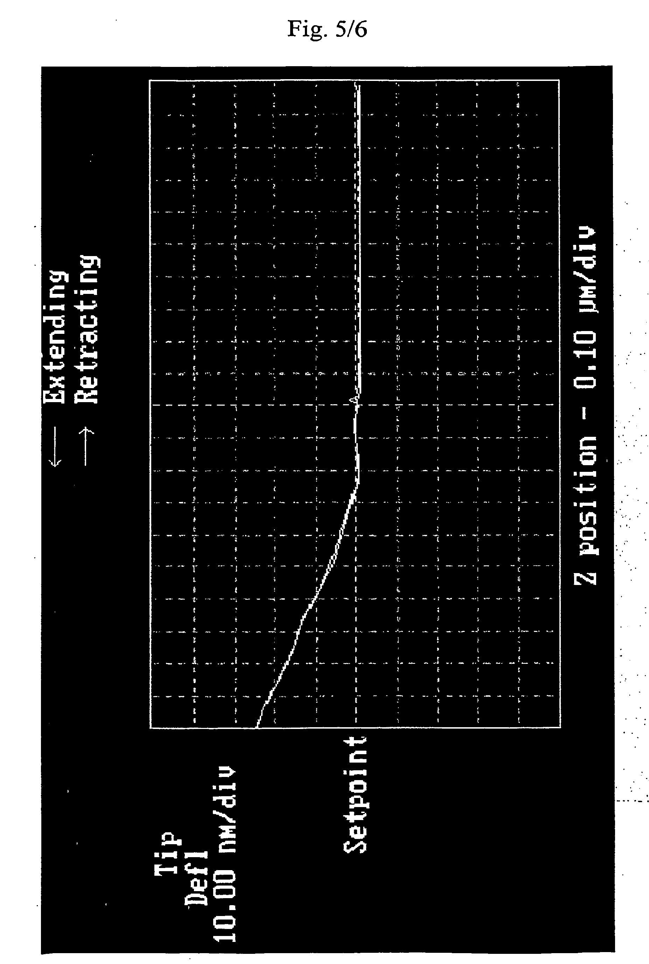

As can be seen from the force-distance profile for this sample (see figure 5), no attractive force was detected on approaching the particle to the coated surface.

There was however a weak and irregular adhesion force on retraction. Probably due to some drag caused by the extension of the fluorinated chain. The drug and cantilever on retraction dragged the chain with them and created what appears to be an attractive force. This force however is mechanical in nature, and is not of the van der Waals type. i.e. its extent stops where the molecular chain finishes, it is a contact force. The system was essentially non attractive and no adhesion energy could be calculated.

The plasma coating is therefore an effective way of reducing particle adhesion.

### Sample 2: aluminium surface cold plasma coated with 1H. 1H, 2H, 2H heptadecafluorodecyl acrylate with a pulsed wave.

The same observation were done on this sample as on sample 1. No attraction is detected, and an irregular adhesion is seen.

Therefore, the 1H, 1H, 2H, 2H heptadecafluorodecyl acrylate coating, either applied as a continuous wave or a pulsed one, is an effective hindrance to particle adhesion to surfaces in a fluorinated environment.

### Sample 3: aluminium surface cold plasma coated with 1H, 1H, 2H, 2H perfluoroctyl acrylate with a continuous wave.

No attraction was detected on approaching the particle to the coated surface. On retraction, no adhesion was observed.

This coating is a very effective way of preventing drug adhesion.

### Sample 4: aluminium surface cold plasma coated with 1H, 1H. 2H, 2H perfluoroctyl acrylate with a pulsed wave.

No attraction was detected on approaching the particle to the coated surface. On retraction, no adhesion was observed.

This coating is a very effective way of preventing drug adhesion.

### Sample 5: aluminium surface cold plasma coated with 1H, 1H, 2H, 2H perfluoroctyl methacrylate with a continuous wave.

No attraction was detected on approaching the particle to the coated surface. On retraction, no adhesion was observed.

This coating is a very effective way of preventing drug adhesion.

### Sample 6: aluminium surface cold plasma coated with 1H, 1H, 2H, 2H perfluoroctyl methacrylate with a plused wave.

No attraction was detected on approaching the particle to the coated surface. On retraction, no adhesion was observed.

This coating is a very effective way of preventing drug adhesion.

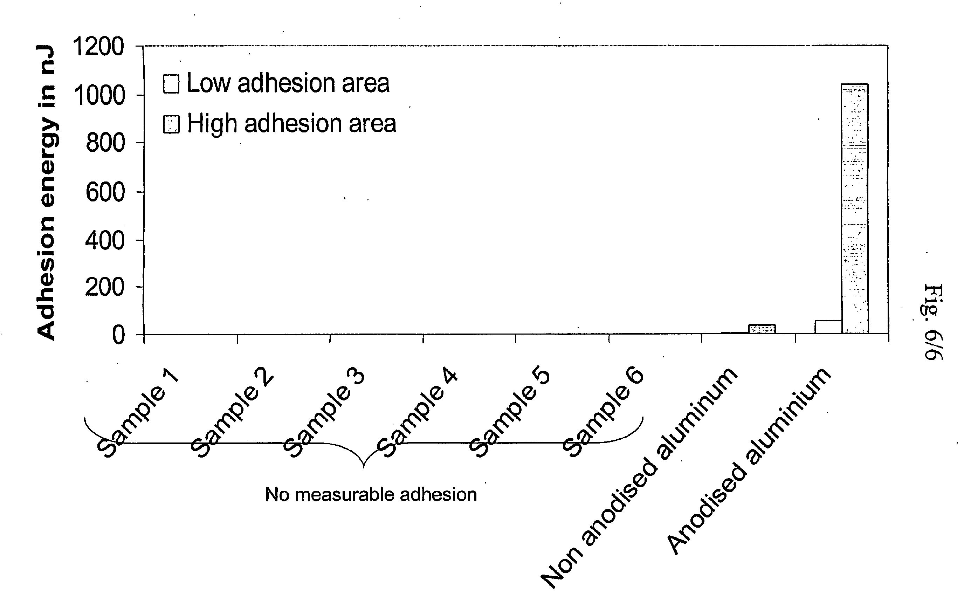

Figure 6 summarises the average energies for all samples. No adhesion energy could be calculated for any of the coated samples, whereas uncoated samples had strong adhesive energies and attractive forces.

### List of figures:

Figure 1: Calculation of adhesion energy from AFM measurements.
Figure 2: Force vs distance curve for FFD against a non anodised aluminium surface in HPFP.
Figure 3: Energy map for the interaction between FFD and a non anodised aluminium surface in HPFP.
Figure 4: Energy map for the interaction between FFD and an anodised aluminium surface in HPFP.
Figure 5: Force vs distance curve for FFD against the surface of sample 1 in HPFP.
Figure 6: Summary of adhesion energies for coated and uncoated samples.

## Claims

1. A device for dispensing a drug by inhalation wherein the device or components thereof where drug adhesion can occur are coated by a cold plasma coating process **characterised in that** the coating is a fluorinated acrylate compound selected from 1H, 1H, 2H, 2H heptadecafluorodecyl acrylate, 1H, 1H, 2H, 2H perfluoroctyl acrylate or 1H, 1H, 2H, 2H perfluoroctyl methacrylate or a mixture thereof.

2. A device according to claim 1 further comprising a can wherein the can is coated.

3. A device according to claim 1 or 2 further comprising a stem wherein the stem is coated.

4. A device according to any one of claims 1 to 3 further comprising an actuator wherein the actuator is coated.

5. A device according to any one of claims 1 to 4 further comprising seals wherein the seals are coated.

6. A device according to any one of claims 1 to 5 further comprising the drug wherein the drug is mometasone, ipratropium bromide, tiotropium and salts thereof, salemeterol, fluticasone propionate, beclomethasone dipropionate, reproterol, clenbuterol, rofleponide and salts, nedocromil, sodium cromoglycate, flunisolide, budesonide, formoterol fumarate dihydrate, budesonide in combination with formoterol), 3-[2-(4-hydruxy-2-oxo-3H-1,3-benzothiazol-7-yl)ethylamino]-N-[2-[2-(4-methylphenyl)elhoxy)ethyl]propansulphonamide, terbutaline, terbutaline sulphate, salbutamol base and sulphate, fenoterol, 3-[2-(4-Hydroxy-2-oxo-3H-1,3-benzothiazol-7-yl)ethylamino]-N-[2-[2-(4-methylphenyl)ethoxy]ethyl]propanesulphonamide, hydrochloride.

## Patentansprüche

1. Gerät zur Verabreichung eines Arzneimittels durch Inhalation, wobei das Gerät oder Komponenten davon, bei denen es zu einem Anhaften von Arzneimitteln kommen kann, durch ein Kaltplasma-Beschichtungsverfahren beschichtet ist/sind, **dadurch gekennzeichnet, daß** es sich bei der Beschichtung um eine fluorierte Acrylatverbindung ausgewählt aus Acrylsäure-1H,1H,2H,2H-heptadecanfluordecylester, Acrylsäure-1H,1H,2H,2H-perfluoroctylester oder Methacrylsäure-1H,1H,2H,2H-perfluoroctylester oder eine Mischung davon handelt.

2. Gerät nach Anspruch 1, weiterhin umfassend eine Dose, wobei die Dose beschichtet ist.

3. Gerät nach Anspruch 1 oder 2, weiterhin umfassend einen Schaft, wobei der Schaft beschichtet ist.

4. Gerät nach einem der Ansprüche 1 bis 3, weiterhin umfassend eine Betätigungsvorrichtung, **dadurch gekennzeichnet, daß** die Betätigungsvorrichtung beschichtet ist.

5. Gerät nach einem der Ansprüche 1 bis 4, weiterhin umfassend Dichtungen, wobei die Dichtungen beschichtet sind.

6. Gerät nach einem der Ansprüche 1 bis 5, weiterhin umfassend das Arzneimittel, wobei es sich bei dem Arzneimittel um Mometason, Ipratropiumbromid, Tiotropium und Salze davon, Salemeterol, Fluticasonpropionat, Beclomethasondipropionat, Reproterol, Clenbuterol, Rofleponid und Salze, Nedocromil, Natriumcromoglycat, Flunisolid, Budesonid, Formoterolfumarat-dihydrat, Budesonid in Kombination mit Formoterol, 3-[2-(4-Hydroxy-2-oxo-3H-1,3-benzothiazol-7-yl)ethylamino]-N-[2-[2-(4-methylphenyl)ethoxy]ethyl]propansulfonamid, Terbutalin, Terbutalinsulfat, Salbutamolbase und -sulfat, Fenoterol, 3-[2-(4-Hydroxy-2-oxo-3H-1,3-benzothiazol-7-yl)ethylamino]-N-[2-[2-(4-methylphenyl)ethoxy]ethyl]propansulfonamidhydrochlorid handelt.

## Revendications

1. Dispositif pour l'administration d'un médicament par inhalation, dans lequel le dispositif ou les composants de celui-ci sur lesquels le médicament peut adhérer sont enduits à l'aide d'un procédé de revêtement par plasma froid **caractérisé en ce que** le revêtement est un composé d'acrylate fluoré choisi parmi l'acrylate de 1H, 1H, 2H, 2H heptadécafluorodécyle, l'acrylate de 1H, 1H, 2H, 2H perfluoroctyle ou le méthacrylate de 1H, 1H, 2H, 2H perfluoroctyle ou un mélange de ceux-ci.

2. Dispositif selon la revendication 1 comprenant en outre une bombe (aérosol), dans lequel la bombe est enduite.

3. Dispositif selon la revendication 1 ou la revendication 2, comprenant en outre une tige, dans lequel la tige est enduite.

4. Dispositif selon l'une quelconque des revendications 1 à 3 comprenant en outre un atomiseur, dans lequel l'atomiseur est enduit.

5. Dispositif selon l'une quelconque des revendications 1 à 4 comprenant en outre des joints d'étanchéité, dans lequel les joints d'étanchéité sont enduits.

6. Dispositif selon l'une quelconque des revendications 1 à 5 comprenant en outre le médicament, dans lequel le médicament est de la mométasone, du bromure d'ipratropium, du tiotropium et des sels de ceux-ci, du salmétérol, du proprionate de fluticasone, du dipropionate de béclométhasone, du reprotérol, du clenbutérol, du rofleponide et ses sels, du nédocromil, du cromoglycate de sodium, du flunisolide, du budésonide, du fumarate de formotérol dihydrate, du budésonide en association avec du formotérol, du 3-[2-4-hydroxy-2-oxo-3H-1,3-benzothiazol-7-yl)éthylamino]-N-[2-[2-(4-méthylphényl)éthoxy)éthyl]propanesulfonamide, de la terbutaline, du sulphate de terbutaline, une base et du sulphate de salbutamol, du fénotérol, du 3-[2-(4-hydroxy-2-oxo-3H-1,3-benzothiazol-7-yl)éthylamino]-N-[2-[2-(4-méthylphényl)éthoxy]éthyl]propanesulfonamide, du chlorhydrate.
